# EUROPEAN PATENT APPLICATION

(11) **EP 4 659 721 A1**
(43) Date of publication of application: **10.12.2025**
(21) Application number: 24305905.2
(22) Date of filing: 07.06.2024
(51) Int. Cl.: A61F 13/08

(54) **LABYRINTH AND ACCORDEON PRESSURE GARMENT**

(71) Applicant: Neuraltide, 75004 Paris (FR)
(72) Inventor: DEGASNE, Benjamin, 75004 Paris (FR); BERTHET, Karine, 75004 Paris (FR)
(74) Representative: Icosa

(57) **Abstract**

Compression slide (10) to be wrapped around a leg of a subject in order to apply a pressure around the leg. The leg of the subject has a back surface (Li) with main apex (A₁) facing any support surface (S). The compression slide comprises an external layer (12) with securing means (14) to secure the compression slide around the leg, a compression pocket to be inflated and being configured to surround the leg in order to apply pressure on both the back and the front surface of the leg. The compression slide comprises one inflatable arrangement to be arranged on the back surface of the leg, with an active zone (to be inflated, a main inactive zone to be located on the main apex of the back surface of the leg and to remain deflated when the compression pocket or the active zone are inflated.

## Description

### FIELD OF INVENTION

The present invention relates to a pressure application garment for applying pressure to at least one part of the body of a subject.

### BACKGROUND OF INVENTION

The "Lower Body Positive Pressure" (LBPP) principle involves applying constant low-pressure levels, that in particular lie in the range 10 to 20 mmHg, to the abdomen and 20-40 mmHg to the legs of a subject, whereby the pressure applied to the abdomen is strictly less than the pressure applied to the legs. This concept was initially developed to ensure an adequate volume distribution in the infra- and supra-aortic areas, in particular for microgravity flight (at high grade, almost ten times higher pressure regimen). LBPP has been documented to displace blood from the lower body to the upper body of the subject, thus increasing the central cardiopulmonary blood volume.

The purpose of the LBPP combination in conjunction with the central unit is to deliver a controlled, constant, homogeneous pressure gradient that can be replicated over the course of sessions for the same patient and also for each patient whatever their morphology.

As mentioned here-above, the pressure regime range for the lower limbs is between 20 and 40 mmHg. These limits define the therapeutic pressure regime: below 20 mmHg the pressure is infra-therapeutic and above 40 mmHg the pressure is supra-therapeutic, deleterious due to the activation of the sympathetic nervous system, which has a systemic effect by reducing cardiac output.

It was realized that surface pressure was not evenly distributed across the lower limbs, with higher surface pressures being recorded at the back of the thighs or calves in the basal state even before inflation pressures were applied. This excess pressure, which could be as high as 15 to 17 mmHg, was maintained when the bags were pressurized, thereby obviating the need for a uniform, controlled pressure regime. This created a sort of undesirable antero-posterior pressure gradient (the pressure gradient being established between the anterior and posterior parts within the lower limbs).

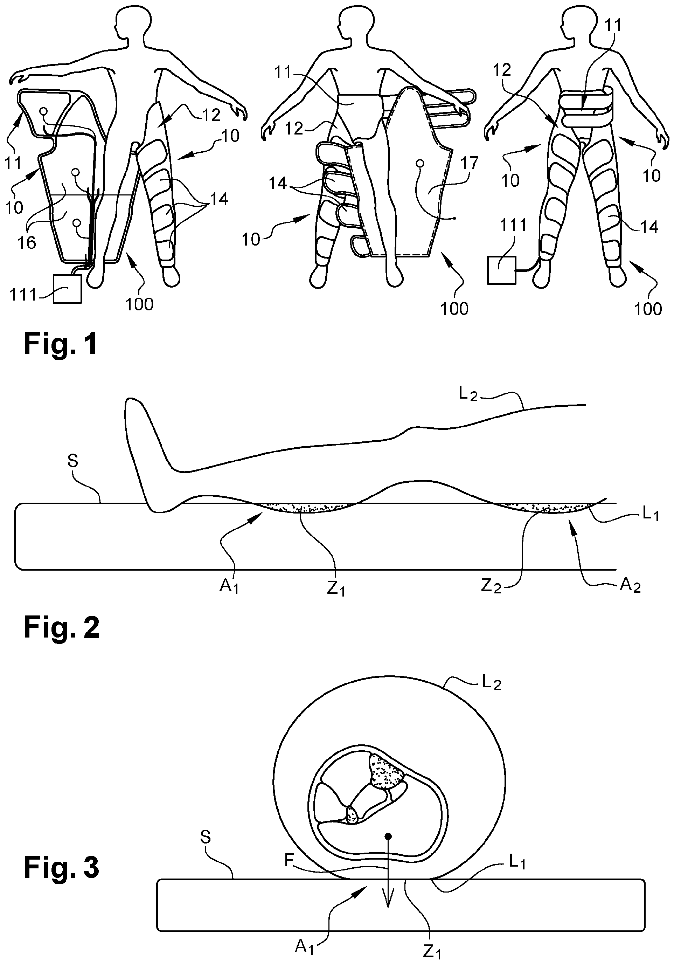

It is essential that the pressure applied to the legs of the subject is homogeneous and equally distributed all around each leg of the subject. It is thus essential to avoid any pressure point generating a localized pressure increase on a spot anywhere over the legs of the subject. This is particularly important for heavier subjects whose legs tend to apply a high pressure on any support surface they are resting on. When a heavier subject thus lays on a support surface (for example a mattress), their legs generate contact zones between a back surface of each leg and the support surface. Those contact zones may generate intense pressure point over and around the contact zones (see figures 2 and 3).

The objective of the present invention is thus to avoid any of those generated pressure points and to enable a homogeneous and even pressure distribution all around each of the legs of the subject, regardless of their size and weight.

### SUMMARY

This invention thus relates to a compression slide configured to be wrapped around a leg of a subject and connected to a pumping device in order to apply a pressure around the leg of the subject, the leg of the subject presenting a back surface and a front surface, the back surface presenting a main apex facing any support surface the subject is laying on, the compression slide comprising:
- an external layer presenting securing means configured to secure the compression slide around the leg of the subject,
- a compression pocket configured to be inflated by means of the pumping device, the compression pocket being configured to surround the leg of the subject when the compression slide is wrapped around the leg in order to apply pressure on both the back and the front surface of the leg,
wherein the compression slide further comprises one inflatable arrangement configured to be arranged on the back surface of the leg, the inflatable arrangement presenting:
- an active zone configured to be connected to the pumping device in order to be inflated,
- a main inactive zone configured to be located on the main apex of the back surface of the leg and to remain deflated when the compression pocket or the active zone are inflated.

This way, the solution enables to reach the here-above mentioned objective. Especially, it enables to provide and easy to use garment which evenly and homogeneously distribute the pression around the leg of the patient, regardless of the size and shape of said leg, regardless of the position of the patient and regardless of the support on which the patient rests.

The system according to the invention may comprises one or several of the following features, taken separately from each other or combined with each other:
- the main inactive zone includes an aperture arranged inside the external layer of the compression slide,
- the main inactive zone further comprises an elastic support layer configured to directly contact the back surface of the leg when the compression slide is wrapped around the leg of the subject,
- the elastic support layer is also part of the main active zone and is configured to directly contact the back surface and the front surface of the leg when the compression slide is wrapped around the leg of the subject,
- the main apex of the back surface of the leg is located on a calf of the subject,
- a secondary apex of the back surface of the leg is located on a thigh of the subject, the inflatable arrangement of the compression slide presenting a secondary inactive zone configured to be located on the secondary apex of the back surface of the leg,
- the compression pocket includes at least partially the active zone of the inflatable arrangement,
- the active zone of the inflatable arrangement comprises a lifting cushion configured to be arranged on an outer surface of the external layer,
- the inflatable arrangement comprises a further secondary inactive zone surrounded by the lifting cushion and arranged to cover a popliteal fossa of the leg of the subject,
- the pressure applied on the subject's leg ranges between 20 and 40mmg, preferably 20mmHg,
- the compression garment is configured to wrap the complete lower body of the subject.

The invention is also about a lower body compression kit comprising a compression garment according to anyone of the preceding technical features, and a pumping device, wherein the pressure applied to the lower body of the subject is higher around the legs of the subject and lower around the abdomen of the subject.

The invention is further about a method for applying pressure to the body of a subject according to a predetermined protocol, including a first predefined pressure value to be applied to the subject's abdomen and a second predefined pressure value to be applied to each of the subject's legs for a predefined duration, using a compression garment according to the description above, said method comprising steps in which:
- each compression slide and the abdomen portion of the compression garment in the deployed configuration is positioned facing the corresponding body part of the subject;
- each compression slide and the abdomen portion of the compression garment in the deployed configuration is placed into an adjusted configuration in which it is adjusted around the corresponding body part of the subject;
- the compression pockets of the compression garment are filled with fluid until a measurement is obtained, for each compression slide and the abdomen portion by activation of the pumping device;
- during the predefined duration, for each compression slide and the abdomen portion, the pressure inside each compression pocket, is maintained at its predetermined pressure value.

The predetermined protocol may be a LBPP treatment, in which:
- the first predefined pressure value to be applied to the subject's abdomen is in the range 10 to 20 mmHg, preferably equal to about 10 mmHg,
- the second predefined pressure value to be applied to each of the subject's lower legs is in the range 20 to 40 mmHg, preferably equal to about 20 mmHg,
- the first predefined pressure value for the abdominal active part is strictly less than the second predefined pressure value for each lower active part,
- the predefined duration is equal to about 90 minutes.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration, purely illustrative and non-limiting examples, with reference to the accompanying drawings:
- **figure 1** is a schematic view of a compression kit comprising a pumping device and a compression garment with a compression slide according to the present invention,
- **figure 2****,** is a schematic side view of a subject's leg around which a compression slide according to the invention is to be wrapped,
- **figure 3****,** is a schematic cross section of said leg,
- **figure 4****,** is an exploded view of the different layers of a first embodiment of the compression slide according to the present invention,
- **figures 5a** and **5b****,** are schematic cross sections of a second and third embodiment of the compression slide according to the present invention,
- **figure 6** is a schematic cross section of a fourth embodiment of the compression slide according to the present invention,
- **figures 7a** and **7b** are exploded view of the different layers of a fifth embodiment according to the present invention,
- **figure 8** is a schematic longitudinal section of a sixth embodiment of the compression slide according to the present invention,
- **figure 9** is a schematic depiction of a fabric strip according to a seventh embodiment of the compression slide according to the present invention,
- **figure 10** is a schematic depiction of the folded strip of figure **9****,**
- **figure 11** is a schematic view from below of the seventh embodiment of the compression slide according to the present invention,
- **figure 12** is a side view of the seventh embodiment of the compression slide according to the present invention.

### DETAILED DESCRIPTION

### Compression garment and compression kit

As can be seen on figure 1, this invention relates to a compression garment 100 comprising an abdomen portion 11 and two compression slides 10. The compression garment 100 is configured to wrap the complete lower body of a subject. Said compression garment 100 is part of a lower body compression kit also comprising a pumping device 111. The pressure applied to the lower body of the subject is higher around the legs of the subject and lower around the abdomen of the subject.

More precisely, the invention is also about a leg compression kit comprising the pumping device 111 and at least one compression slide 10 of the kind that will be described further below. The pressure applied on the subject's leg(s) ranges between 20 and 40mmg, preferably 20mmHg.

The pumping device 111 is preferably a gas pumping device, more particularly an air pumping device. The compression slide 10 and the compression garment 100 are thus preferably inflated by means of gas and more particularly by means of air. However, they could be inflated by means of a liquid, for example water.

### Compression slide

As can be seen on figure 1, each compression slide 10, according to the present invention, is configured to be wrapped around a leg of a subject. Each compression slide 10 is further configured to be connected to the pumping device 111 in order to be inflated ant to apply a homogeneous pressure P around the leg of the subject.

As can be seen on figures 2 and 3, each leg of the subject presents a back surface Li and a front surface L₂. The back surface Li presents a main apex A₁ and faces any support surface S the subject may be laying on. In the present invention, the term "apex" is understood to be a convex curve extending outwards with regards of the muscle mass of the leg. It is thus a zone of the leg which exerts a high pressure F (or force) on the support surface S when the subject is laying on. Such a support surface S could be a mattress, for example. More particularly, the main apex A₁ of the leg could be the calf of the subject. The back surface Li of the leg may present a secondary apex A₂. Particularly for heavier subjects. More particularly, this secondary apex A₂ could be the thigh of the subject. Any apex A₁, A₂ of the back surface Li generates a contact zone Zi with the support surface S over which and around which pressure points are generated.

As can be seen on figure 1, the compression slide 10 comprises:
- an external layer 12 presenting securing means 14 configured to secure the compression slide 10 around the leg of the subject,
- a compression pocket 16 configured to be inflated by means of the pumping device 111,
- preferably, an inner layer 17 soft and flexible enough not to hurt the patient and wrap the leg smoothly following the curves of the leg.

The inner layer 17 preferably presents a three layers structure presenting specific elastic and extension properties as presented in application EP 19 306 512.

The external layer 12 presents an outer surface and an inner surface. In some embodiment, the outer surface is made of a multitude of tiny loops that allow for a hook strap counterpart to be hold onto, like a Velcro Scratch^{®} (see figure 1). In this embodiment, the securing means 14 can be secured anywhere on the outer surface of the external layer 12. In some other embodiments, the securing means 14 can for example comprise clips, belts, laces, etc.

The external layer 12 is preferably secured to the compression pocket 16. The external layer 12 may form a wall of the compression pocket 16. In some embodiments, the compression pocket 16 is separate and independent from the external layer 12, in order to enable a better adaptation of the slide 10 to the anatomy of the patient, once the slide 10 is wrapped around the leg of the patient. In some alternative embodiment, the pocket 16 and the external layer 12 are strongly connected to each other, for example by means of a peripherical sewing path.

The compression pocket 16 is configured to surround the leg of the subject when the compression slide 10 is wrapped around said leg. The compression pocket 16 is thus configured to apply pressure P, preferably the same pressure P, on both the back and the front surfaces L₁, L₂ of the leg.

The compression pocket 16 presents at least two walls, an external wall 16a and an internal wall 16b which are for example sewn (and taped) or welded together. The compression pocket is made out of 2 airtight materials (coated fabrics, films or membranes) that allow for them to be welded together thus creating an airtight bladder.

The compression slide 10 further comprises one inflatable arrangement 18 configured to be arranged on a partial or total surface, preferably the back surface Li of the leg. This inflatable arrangement 18 can present several different shapes, sizes and realizations depending on the embodiments of the compression slide 10. This inflatable arrangement 18 will be described further below.

Regardless of the considered embodiment, the inflatable arrangement 18 presents:
- an active zone 20 configured to be connected to the pumping device 111 in order to be inflated,
- a main inactive zone 22 configured to be located, when the compression slide 10 is wrapped around the subject's leg, on the main apex A₁ of the back surface Li of the leg, and to remain deflated no matter what, especially when the compression pocket 16 or the active zone 20 are inflated.

In some embodiments, the inflatable arrangement 18 can present several active zones 20 located on different parts of the compression slide 10.

The active zone 20 can be located at different places of the compression slide 10 and can present different shapes. Regardless of its location or shape, the active zone 20 is configured to regulate and control the movements and repartition of the fluid inside the compression pocket 16 when the compression pocket 16 and the active zone 20 are inflated. This control of the fluid movements and repartition contributes to avoid the generation of pressure points and thus contributes to even out and homogenize the pressure P inside the compression pocket 16, all around the leg of the subject.

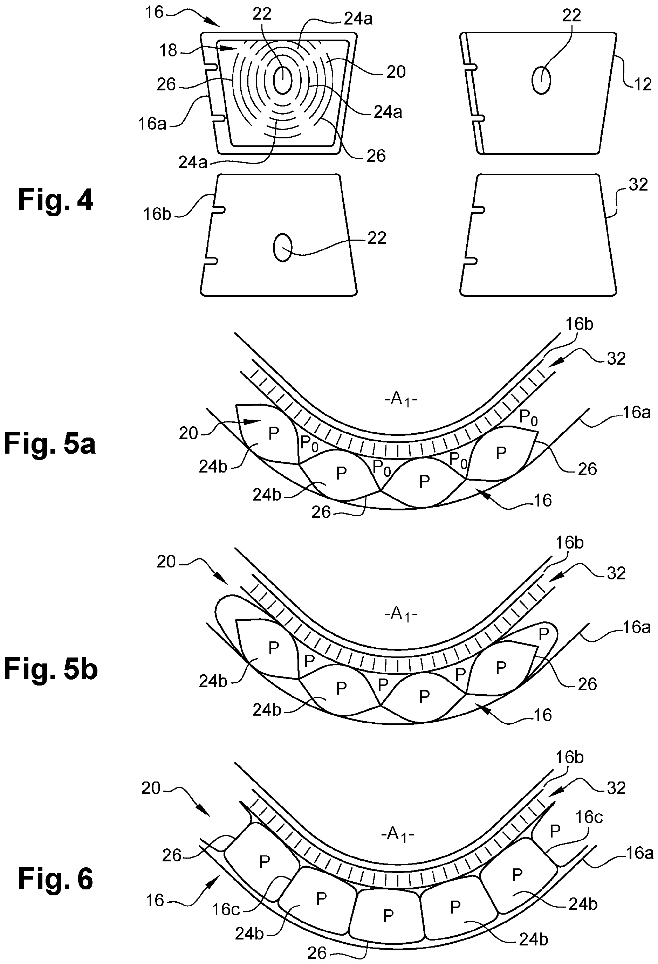

In some embodiments, for example depicted in figures 4, 5a, 5b, 6, 7a, 7b, 8 and 11, the compression pocket 16 includes at least partially the active zone 20 of the inflatable arrangement 18. In some embodiments, the compression pocket 16 includes the complete inflatable arrangement 18 if it presents only one active zone 20.

More particularly, as can be seen on figure 4, the inflatable arrangement 18 comprises a series of interconnected compartments 24a arranged inside and connected to the compression pocket 16. Those interconnected compartments 24a define a series of fluidic pathways which organize and canalize the fluidic movements inside the compression pocket 16 when the compression pocket 16 is inflated. The interconnected compartments 24a are generated by connecting the two opposite walls 16a, 16b of the compression pocket 16 by welding or sewing them together along a series of connection lines 26. In some embodiments, (see figures 5a and 5b). the two opposite walls 16a, 16b are directly connected to each other. In some alternative embodiment (see figure 6), the two opposite walls 16a, 16b are indirectly connected together, by welding or sewing a series of spacers 16c of a given height between them. Said spacers 16c are pieces of fabric and their height may vary depending on their location. Those connection lines 26 define a pattern. Said pattern can be symmetrically arranged around the main inactive zone 22, as can be seen in figure 4.

In alternative embodiments, as can be seen on figures 5a, 5b and 6, the inflatable arrangement 18 comprises a series of interconnected isolated compartments 24b arranged inside the compression pocket 16. The compartments 24b are connected to each other, and the series of isolated compartment 24b is tightly isolated from the residual space inside the compression pocket 16 and is to be inflated/deflated independently from the compression pocket 16 (see figures 5a, 5b). The pressure inside the inflatable arrangement 18 can be the same than the pressure inside the compression pocket 16 (see figure 5b). The pressure inside the inflatable arrangement 18 can be different than the pressure inside the compression pocket 16 (see figure 5a). In some alternative embodiment, the isolated compartments 24 occupy the complete space inside the compression pocket 16 (see figure 6). Regardless of the embodiment, the isolated interconnected compartments 24b define a series of fluidic pathways which organize and canalize the fluidic movements inside the compression pocket 16. This prevents, once the compression slide 10 is inflated, the fluid to leave a high pressure area (generated by a contact zone Zi between the support surface S and any apex A₁, A₂ of the back surface Li of the leg) and to gather in a lower pressure area. The inflatable arrangement 18 thus maintains the fluid equally distributed inside the compression pocket 16 and thus counter-balance any pressure points. The isolated interconnected compartments 24b are generated by connecting the two opposite walls 16a, 16b of the compression pocket 16 by welding or sewing them together along a series of connection lines 26.

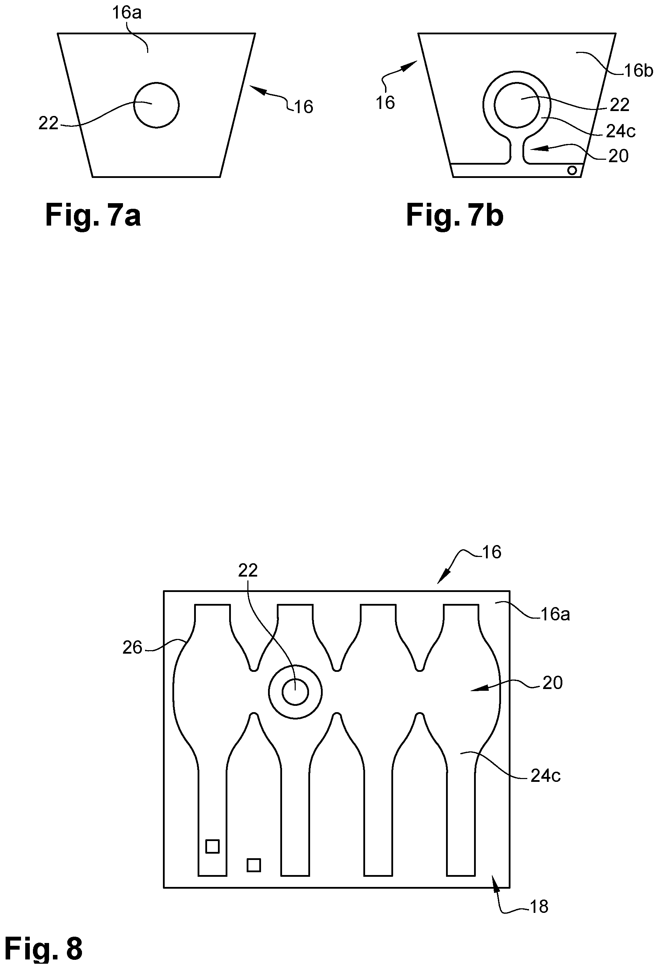

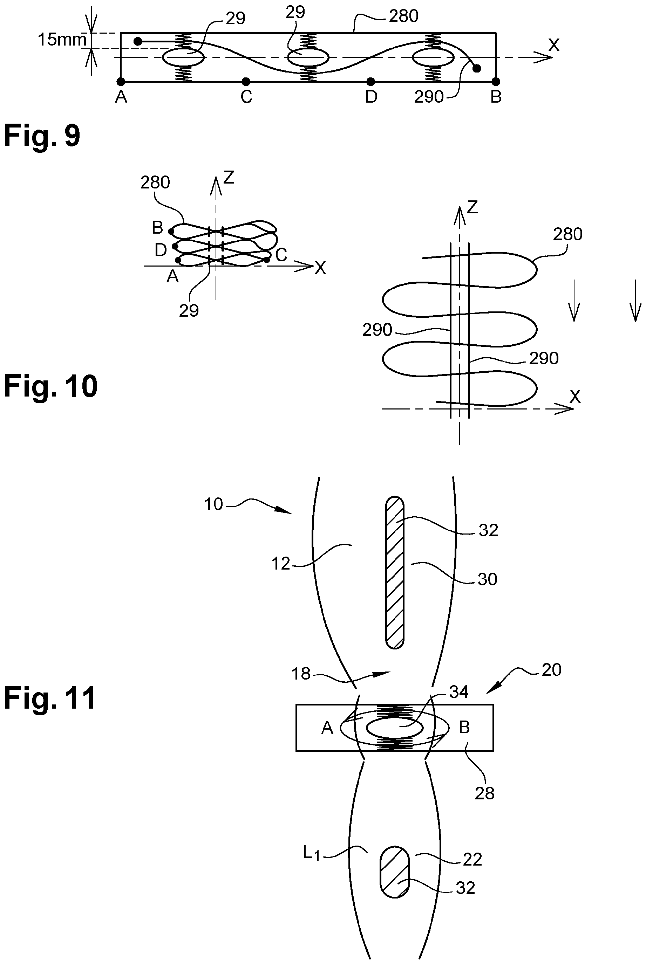

In others embodiments, as illustrated in figures 7a, 7b, and 9, the inflatable arrangement 18 comprises one isolated compartment 24c arranged inside the compression pocket 16. The isolated compartment 24c is tightly isolated from the residual space inside the compression pocket 16 and is to be inflated/deflated independently from the compression pocket 16. Preferably, as can be seen on figures 7a, 7b, and 8, the isolated compartment 24c forms a fluidic path surrounding the main inactive zone 22 extending within the compression pocket 16. The one isolated compartment 24c are generated by connecting the two opposite walls 16a, 16b of the compression pocket 16 by welding or sewing them together along a series of connection lines 26. This way, around the main inactive zone 22 and more particularly around the apexes A₁, A₂ of the back surface Li of the leg, the compression slide generates localized higher-pressure pathways which counterbalance the potential pressure points generated by any contact zone Z₁ between the support surface S and any apex A₁, A₂ of the back surface Li of the leg.

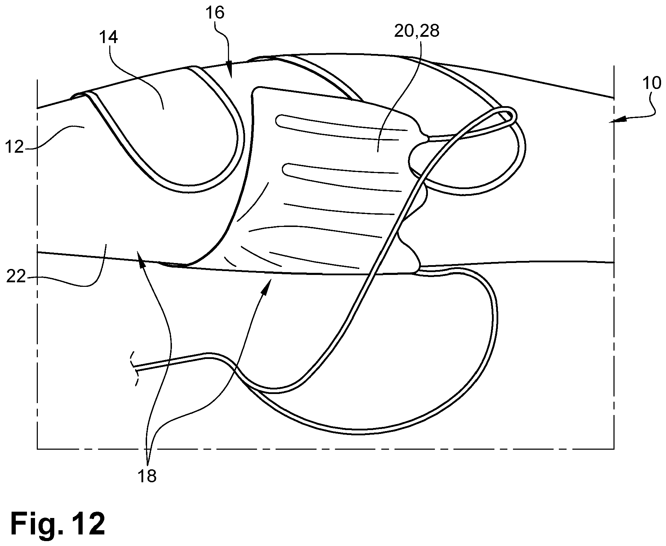

In the embodiment depicted in figures 9, 10, 11 and 12, the active zone 20 of the inflatable arrangement 18 comprises a lifting cushion 28 configured to be arranged on the outer surface of the external layer 12. In some unshown embodiment, the lifting cushion 28 could be arranged inside the external layer 12. The lifting cushion 28 is made of at least two fabric strips 280 extending along a longitudinal axis X presenting a series of openings 29 aligned with a said longitudinal axis X. The two fabric strips 280 are secured to each other (welded or sewn together) in order to create an isolated pocket which can be inflated. The two secured fabric strips 280 are then folded as depicted in figure 10 in order to align the openings 29 along a stacking axis Z perpendicular to the longitudinal axis Z. The size and the shape of the openings 29 is designed to occupy a large space inside the strips 280 and to, preferably, leave only 13 to 16mm of fabric on the edges (see figure 9, black marked zones). This way, once the lifting cushion 280 is inflated, fluid can circulate around the openings 29 but regarding the very small surface, very little pressure is exerted on the leg of the patient. A string 290 is inserted inside the pocket delimited by the two fabric strips 280 in order to ease the deflation of the lifting cushion once it has been inflated. The technical advantage of such a folded lifting cushion over a simple inflated cushion (with same inflation volume) is the applied pressure to cushion volume ratio. The specific shape of the lifting cushion 28 according to the present invention multiplies the volumes and thus enables to generate an important lifting force and leverage height to the leg of the subject while limiting the applied pressure on said leg (see further below). The lifting cushion 28 further enables to block the leg in place, which can be an advantage as the applied treatment can last about 90 minutes. Position fatigue is an important drawback of the treatment, and a lifting cushion 28 according to the invention may help against it. Such a lifting cushion 28 improves the comfort of the subject during treatment.

The lifting cushion 28 is configured to be located under the knee of the subject, more particularly under the popliteal fossa of the subject. Depending on the size of the subject, each fabric strip 280 present a length ranging from 1300mm and 1800mm and a width ranging from 130 to 160mm.

The main inactive zone 22, on the other hand, works as a "pressure release area" in order to lower the pressure, inside the compression pocket 16, at specific places where it would naturally rise due to the specific shape of the leg. In particular, around and over the main and secondary apexes A₁, A₂ of the leg, when they form contact zones Z₁, Z₂ with the support surface S. As this main inactive zone 22 remains deflated, no fluid can enter it when the compression slide 10 is inflated and the pressure is automatically reduce, with regards to the surrounding inflated elements as the compression pocket 16 or the active zone 20 of the inflatable arrangement 18. The main inactive zone 22 presents dimensions ranging from 30cm2 to 1000cm2 in order to cover at least the tip of the main and secondary apexes A₁, A₂ and possibly the whole leg of the patient. It preferably presents an egg shaped shape in order to best fit the natural shape of a human leg, more particularly a human calf.

Concretely, the main inactive zone 22 is preferably a zone tightly separated from the inside of the compression pocket 16, arranged inside the compression pocket 16. It may also be arranged inside the external layer 12 of the compression slide 10.

As can be seen on figures 4, 5 and 6a, 6b, in some embodiments, the main inactive zone 22 includes a zone of the compression pocket 16 in which the two opposites walls of the compression pocket 16 are sewn or soldered together in order to generate an isolated zone inside said compression pocket 16. In some embodiments, the main inactive zone 22 includes an aperture arranged inside the external layer 12 of the compression slide 10. This aperture avoids residual pressure or tensions from the fabric over the apex A₁ of the leg. The main inactive zone 22 may include an aperture arranged inside a wall of the compression pocket 16. The main inactive zone 22 may include two apertures arranged inside the first wall and the second wall of the compression pocket 16.

It is well known that an easy way to ease tensions and avoid pressure points is to use an elastic fabric. In some embodiments, the aperture arranged in the external layer 12 of the wall of the compression pocket 16 is closed by means of an elastic support layer 32, more precisely, a piece of elastic fabric. The main inactive zone 22 thus comprises an elastic support layer 32 configured to directly contact the back surface Li of the leg when the compression slide 10 is wrapped around the leg of the subject. The main inactive zone 22 is thus an (at least partially) elastic relaxation zone.

In some embodiments, the elastic support layer 32 is also part of the main active zone 20 and is configured to directly contact the back surface Li and the front surface L₂ of the leg when the compression slide 10 is wrapped around the leg of the subject.

In some embodiments, as can be seen on figure 11, the inflatable arrangement 18 of the compression slide 10 comprises a secondary inactive zone 30 configured, when the compression slide is wrapped around the subject's leg, to be located on the secondary apex A₂ of the back surface Li of the leg. In particular, said secondary inactive zone 30 is configured to be located over the back surface of the thigh of the subject. In some particularly large embodiments, further secondary inactive zones 30 may be located under the popliteal fossa (below the knee) of the patient to ensure the absence of over-pressure. Further secondary inactive zones 30 me thus be located at different places around the patient's leg. Any secondary inactive zone 30 may present, similarly to the main inactive zone 22, elastic properties, in order to form an elastic relaxation zone.

In the embodiment including a lifting cushion 28, the inflatable arrangement 18 comprises a further secondary inactive zone 34 surrounded by the lifting cushion 28 and arranged to cover the popliteal fossa (below the knee) of the leg of the subject (see figure 11). Due to the design of the lifting cushion 28, and the specific positioning of the openings 29 inside the fabric strips 280, the pression applied around the openings 29 once the lifting cushion is inflated is minimal. The stacking of the openings 29 creates a this further secondary inactive zone 34 appears while the lifting cushion 28 is being inflated. Its purpose is to enable the lifting of the subject's leg without generating new pressure points on the back surface Li of the leg of the subject.

### Method

The slide 10 and garment 100 described here-above enable to carry out a method for applying pressure to the body of a subject according to a predetermined protocol. This method includes a first predefined pressure value to be applied to the subject's abdomen and a second predefined pressure value to be applied to each of the subject's legs for a predefined duration, using a compression garment 100 comprising compression slides 10 as described above. Said method comprising steps in which:
- each compression slide 10 and the abdomen portion A of the compression garment 100 in the deployed configuration is positioned facing the corresponding body part of the subject;
- each compression slide 10 and the abdomen portion A of the compression garment 100 in the deployed configuration is placed into an adjusted configuration in which it is adjusted around the corresponding body part of the subject;
- the compression pockets 16 of the compression garment 100 are filled with fluid until a measurement is obtained, for each compression slide 10 and the abdomen portion A by activation of the pumping device 111;
- during the predefined duration, for each compression slide 10 and the abdomen portion A, the pressure inside each compression pocket 16, is maintained at its predetermined pressure value.

The predetermined protocol is a LBPP treatment, in which:
- the first predefined pressure value to be applied to the subject's abdomen A is in the range 10 to 20 mmHg, preferably equal to about 10 mmHg,
- the second predefined pressure value to be applied to each of the subject's lower legs is in the range 20 to 40 mmHg, preferably equal to about 20 mmHg,
- the first predefined pressure value for the abdominal active part is strictly less than the second predefined pressure value for each lower active part,
- the predefined duration is equal to about 90 minutes.

The present invention describes the means used to counteract the effects of gravity on the distribution of the surface pressure regime over the posterior part (back surface Li) of the lower limbs (legs) of the subject. The means described make it possible to have a uniform surface pressure regime all around the lower limbs (thigh and calf) whatever the morphology of the subject (adaptation of the shapes of the inactive zone 22 according to the sizes of the subjects, adaptation of the size of the lifting cushion 28 according to the sizes of the subject). Having a well-distributed air cushion around the thigh and calf muscle masses of the leg of the subject is crucial to achieving the expected physiological effects (these functions are also described as essential in our regulatory dossier).

## Claims

1. Compression slide (10) configured to be wrapped around a leg of a subject and connected to a pumping device (111) in order to apply a pressure around the leg of the subject, the leg of the subject presenting a back surface (L₁) and a front surface (L₂), the back surface (L₁) presenting a main apex (A₁) facing any support surface (S) the subject is laying on, the compression slide (10) comprising:
- an external layer (12) presenting securing means (14) configured to secure the compression slide (10) around the leg of the subject,
- a compression pocket (16) configured to be inflated by means of the pumping device (111), the compression pocket (16) being configured to surround the leg of the subject when the compression slide (10) is wrapped around the leg in order to apply pressure on both the back (L₁) and the front surface (L₂) of the leg,
**wherein** the compression slide (10) further comprises one inflatable arrangement (18) configured to be arranged on the back surface (L₁) of the leg, the inflatable arrangement (18) presenting:
- an active zone (20) configured to be connected to the pumping device (111) in order to be inflated,
- a main inactive zone (22) configured to be located on the main apex (A₁) of the back surface (L₁) of the leg and to remain deflated when the compression pocket (16) or the active zone (20) are inflated.

2. Compression slide (10) according to the preceding claim, **wherein** the main inactive zone (22) includes an aperture arranged inside the external layer (12) of the compression slide (10).

3. Compression slide (10) according to any one of the preceding claims, **wherein** the main inactive zone (22) further comprises an elastic support layer (32) configured to directly contact the back surface (L₁) of the leg when the compression slide (10) is wrapped around the leg of the subject.

4. Compression slide (10) according to the preceding claim, **wherein** the elastic support layer (32) is also part of the main active zone (20) and is configured to directly contact the back surface (L₁) and the front surface (L₂) of the leg when the compression slide (10) is wrapped around the leg of the subject.

5. Compression slide (10) according to any one of the preceding claims, **wherein,** the main apex (A₁) of the back surface (L₁) of the leg is located on a calf of the subject.

6. Compression slide (10) according to the preceding claim, **wherein** a secondary apex (A₂) of the back surface (L₁) of the leg is located on a thigh of the subject, the inflatable arrangement (18) of the compression slide (10) presenting a secondary inactive zone (30) configured to be located on the secondary apex (A₂) of the back surface (L₁) of the leg.

7. Compression slide (10) according to any of the preceding claims, **wherein** the compression pocket (16) includes at least partially the active zone (20) of the inflatable arrangement (18).

8. Compression slide (10) according to any of the preceding claims, **wherein** the active zone (20) of the inflatable arrangement (18) comprises a lifting cushion (28) configured to be arranged on an outer surface of the external layer (12).

9. Compression slide (10) according to the precedent claim, **wherein** the inflatable arrangement (18) comprises a further secondary inactive zone (34) surrounded by the lifting cushion (28) and arranged to cover a popliteal fossa of the leg of the subject.

10. Leg compression kit comprising a pumping device (111) and a compression slide (10) according to any one of the preceding claims, **wherein** the pressure applied on the subject's leg ranges between 20 and 40mmg, preferably 20mmHg.

11. Compression garment (100) comprising an abdomen portion (A) and two compression slides (10) according to any one of the preceding claims, **wherein** the compression garment (100) is configured to wrap the complete lower body of the subject.

12. Lower body compression kit comprising a compression garment (100) according to the preceding claim and a pumping device (111), **wherein** the pressure applied to the lower body of the subject is higher around the legs of the subject and lower around the abdomen of the subject

13. Method for applying pressure to the body of a subject according to a predetermined protocol, including a first predefined pressure value to be applied to the subject's abdomen and a second predefined pressure value to be applied to each of the subject's legs for a predefined duration, using a compression garment (100) according to claims 11 or 12, said method comprising steps in which:
- each compression slide (10) and the abdomen portion (A) of the compression garment (100) in the deployed configuration is positioned facing the corresponding body part of the subject;
- each compression slide (10) and the abdomen portion (A) of the compression garment (100) in the deployed configuration is placed into an adjusted configuration in which it is adjusted around the corresponding body part of the subject;
- the compression pockets (16) of the compression garment (100) are filled with fluid until a measurement is obtained, for each compression slide (10) and the abdomen portion (A) by activation of the pumping device (111);
- during the predefined duration, for each compression slide (10) and the abdomen portion (A), the pressure inside each compression pocket (16), is maintained at its predetermined pressure value.

14. Method for applying pressure according to the preceding claim, wherein the predetermined protocol is a LBPP treatment, in which:
- the first predefined pressure value to be applied to the subject's abdomen (A) is in the range 10 to 20 mmHg, preferably equal to about 10 mmHg,
- the second predefined pressure value to be applied to each of the subject's lower legs is in the range 20 to 40 mmHg, preferably equal to about 20 mmHg,
- the first predefined pressure value for the abdominal active part is strictly less than the second predefined pressure value for each lower active part,
- the predefined duration is equal to about 90 minutes.
